# EUROPEAN PATENT APPLICATION

(11) **EP 3 836 158 A1**
(43) Date of publication of application: **16.06.2021**
(21) Application number: 19845996.8
(22) Date of filing: 26.07.2019
(51) Int. Cl.: G16H 50/30, A61B 5/0205

(54) **DOCTOR HEALTH MONITORING SYSTEM BASED ON MEDICAL APPARATUS**

(30) Priority: 07.08.2018 CN 201810893040
(71) Applicant: Wuhan Youcare Technology Co., Ltd., Wuhan, Hubei 430223 (CN)
(72) Inventor: WANG, Shaogang, Wuhan, Hubei 430223 (CN); ZHOU, Xianghua, Wuhan, Hubei 430223 (CN); LONG, Xueping, Wuhan, Hubei 430223 (CN); CHENG, Shuai, Wuhan, Hubei 430223 (CN); LI, Ying, Wuhan, Hubei 430223 (CN); LONG, Gang, Wuhan, Hubei 430223 (CN); WU, Shuxiang, Wuhan, Hubei 430223 (CN); LIU, Chengpeng, Wuhan, Hubei 430223 (CN); ZHANG, Yuan, Wuhan, Hubei 430223 (CN); MAO, Yeyun, Wuhan, Hubei 430223 (CN)
(74) Representative: Hellmich, Wolfgang
(86) International application number: PCT/CN2019/097914
(87) International publication number: WO 2020/029814

(57) **Abstract**

A doctor health monitoring system based on a medical apparatus includes the medical apparatus (1), a detection system (2), a control system (3) and an artificial intelligence system (14). The detection system (2) is configured to detect health indicators of doctors and send the detected health indicators to the control system (3). The control system (3) and the artificial intelligence system (14) are integrated within the medical apparatus (1). The control system (3) is configured to send the detected health indicators from the detection system to a display device (11) for display. The artificial intelligence system (14) is configured to analyze the detected health indicators in the cloud server (12) for obtaining a diagnostic accuracy of the detected health indicators and a physical health status of the doctors. The detection system (2) capable of detecting the health indicators of the doctors is connected with the medical apparatus (1).

## Description

### BACKGROUND OF THE PRESENT INVENTION

### Field of Invention

The present invention relates to the field of medical equipment technology, and more particularly to a doctor health monitoring system based on a medical apparatus.

### Description of Related Arts

In recent years, the health of doctors has been ignored by society and even doctors themselves. Especially in the long-term work, doctors pay more attention to patients and are more likely to ignore their own health problems. They deal with patients almost every day. Each clinical diagnosis and treatment is a rigorous process of thinking activity for them, which is easily affected by emotional and psychological conditions. A slight loss may lead to missed diagnosis, misdiagnosis, surgical operation errors and other serious consequences, thereby affecting the service quality of doctors and the treatment effect of patients.

### SUMMARY OF THE PRESENT INVENTION

An object of the present invention is to solve the shortcomings of the above-mentioned background technology and provide a doctor health monitoring system based on a medical apparatus, which is able to monitor health indicators of doctors during diagnosis and treatment in real time, and predict the physical health status of the doctors. When a certain health indicator exceeds a forewarning value, a warning device automatically sends an alarm to better protect the health of doctors.

Accordingly, the present invention adopts technical solutions as follows. A doctor health monitoring system based on a medical apparatus comprises the medical apparatus, a detection system, a control system and an artificial intelligence system, wherein the detection system is configured to detect health indicators of doctors and send the detected health indicators to the control system; the control system and the artificial intelligence system are integrated within the medical apparatus; the control system is configured to send the detected health indicators from the detection system to a display device for display and transmit the detected health indicators to a cloud server for storage; the artificial intelligence system is configured to analyze the detected health indicators in the cloud server for obtaining a diagnostic accuracy of the detected health indicators and predicting health conditions of the doctors.

Preferably, the detection system comprises a pulse sensor module, a blood pressure sensor module, a heart rate sensor module, a body temperature sensor module, and an ECG (electrocardiogram) sensor module, wherein the pulse sensor module, the blood pressure sensor module, the heart rate sensor module, the body temperature sensor module, and the ECG sensor module are configured to detect pulse monitoring data, blood pressure monitoring data, heart rate monitoring data, body temperature monitoring data, and ECG monitoring data of the doctors, respectively.

Preferably, the detection system is embedded into the medical apparatus and is configured to collect the health indicators of the doctors through the pulse sensor module, the blood pressure sensor module, the heart rate sensor module, the body temperature sensor module, and the ECG sensor module.

Preferably, the control system comprises an MCU (micro controller unit), a main control module and a display module, wherein the MCU is configured to read the detected health indicators detected by the detection system in real time and send the detected health indicators to the main control module; the main control module is configured to display the detected health indicators on the display device through the display module after receiving the detected health indicators.

Preferably, the control system further comprises a communication module, wherein after reading the detected health indicators, the MCU sends the detected health indicators to the cloud server through the communication module for storage, and logs in to the cloud server to view the detected health indicators.

Preferably, the control system further comprises a printer drive module, wherein after receiving the detected health indicators, the main control module sends the detected health indicators to a print device for printing through the printer drive module.

Preferably, the control system further comprises a forewarning module, wherein after receiving the detected health indicators, the main control module compares the detected health indicators with forewarning data, an alarm is sent through the forewarning module when the detected health indicators exceed a range of the forewarning data.

Preferably, the control system further comprises a storage module for storing the detected health indicators received by the main control module.

According to the present invention, the detection system capable of detecting the health indicators of the doctors is connected with the medical apparatus. The doctor health monitoring system provided by the present invention is able to detect the health indicators of the doctors in real time, know the physical health conditions of the doctors, and promptly resolve hypoglycemia and other situations of the doctors during the diagnosis process, so as to ensure smooth medical process.

### BRIEF DESCRIPTION OF THE DRAWINGS

Fig. 1 is a block diagram of a doctor health monitoring system based on a medical apparatus of the present invention.
Fig. 2 is a block diagram of a control system and a detection system of the present invention.

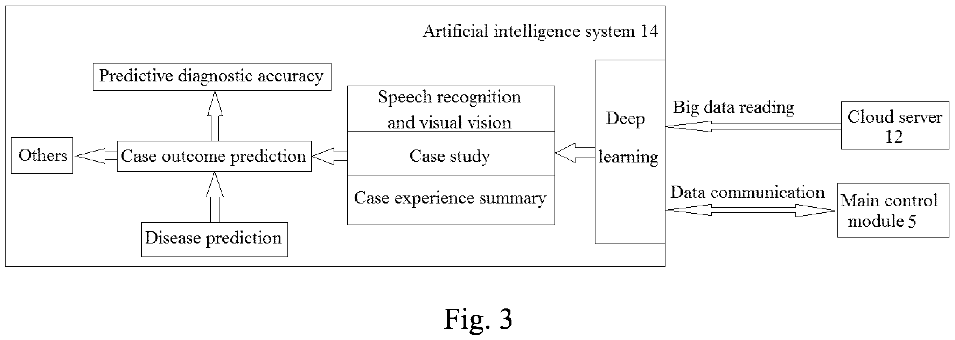

Fig. 3 is a block diagram of an artificial intelligence system of the present invention.

In the drawings, 1: medical apparatus; 2: detection system; 3: control system; 4: MCU (micro controller unit); 5: main control module; 6: display module; 7: communication module; 8: printer drive module; 9: forewarning module; 10: storage module; 11: display device; 12: cloud server; 13: print device; 14: artificial intelligence system.

### DETAILED DESCRIPTION OF THE PREFERRED EMBODIMENT

The present invention is further explained in detail with the drawings and embodiments to be better understood, but these drawings and embodiments are not the limitation to the present invention.

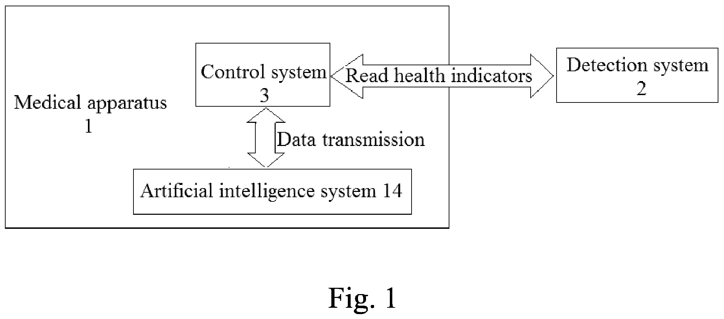

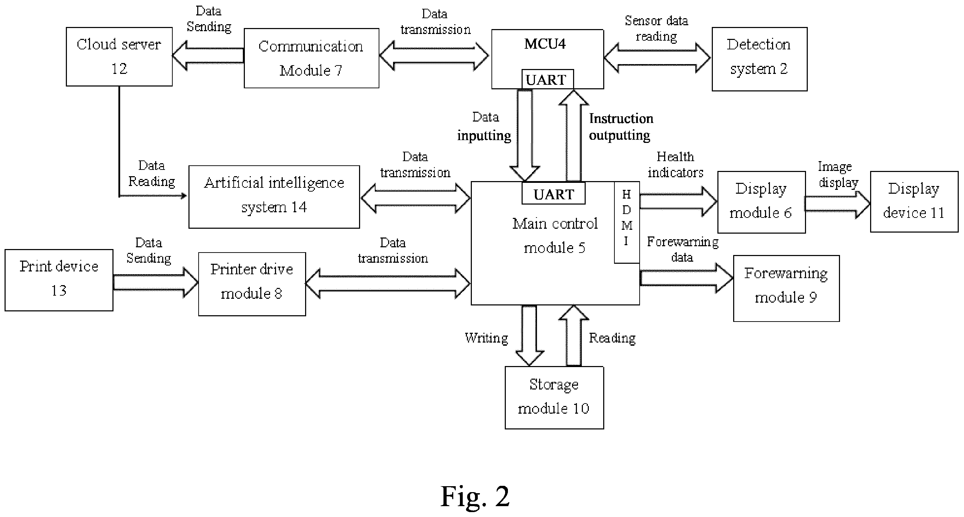

As shown in Figs. 1 and 2, a doctor health monitoring system based on a medical apparatus according to a preferred embodiment of the present invention is illustrated, which comprises the medical apparatus 1, a detection system 2, a control system 3 and an artificial intelligence system 14, wherein the detection system 2 is configured to detect health indicators of doctors and send the detected health indicators to the control system 3; the control system 3 and the artificial intelligence system 14 are integrated into the medical apparatus 1; the control system 3 is configured to send the detected health indicators from the detection system 2 to a display device 11 for display and transmit the detected health indicators to a cloud server 12 for storage; the artificial intelligence system 14 is configured to perform deep learning, speech recognition, machine vision, case study and case experience summary on case data in the cloud server 12, so as to quickly and accurately obtain a diagnostic accuracy of the detected health indicators, and at the same time, analyze and research the detected health indicators for predicting health conditions of the doctors, such as heart disease. The doctor health monitoring system provided by the present invention is not only limited to the field of medical equipment, but also is able to be applied to fields with high-intensity work such as aviation, automobile, and office.

Preferably, the detection system 2 comprises a pulse sensor module, a blood pressure sensor module, a heart rate sensor module, a body temperature sensor module, an ECG (electrocardiogram) sensor module, and a fatigue sensor module. Of course, the detection system is not limited to only including these sensor modules, and it is able to include any sensor module that collects physical health indicators. The pulse sensor module, the blood pressure sensor module, the heart rate sensor module, the body temperature sensor module, the ECG sensor module, and the fatigue sensor module are configured to detect pulse monitoring data, blood pressure monitoring data, heart rate monitoring data, body temperature monitoring data, ECG monitoring data and fatigue monitoring data of doctors, respectively. When the detection system includes other sensor modules, other physical health data are able to be monitored.

Preferably, the detection system 2 is embedded into the medical apparatus 1 and is configured to collect the health indicators of the doctors through the pulse sensor module, the blood pressure sensor module, the heart rate sensor module, the body temperature sensor module, the ECG sensor module, and the fatigue sensor module.

Preferably, the control system 3 comprises an MCU (micro controller unit) 4, a main control module 5 and a display module 6, wherein the MCU 4 is configured to read the detected health indicators detected by the detection system in real time and send the detected health indicators to the main control module 5; the main control module 5 is configured to display the detected health indicators on the display device 11 through the display module 6 after receiving the detected health indicators.

Preferably, the control system 3 further comprises a communication module 7, wherein after reading the detected health indicators, the MCU 4 sends the detected health indicators to the cloud server 12 through the communication module 7 for storage, and logs in to the cloud server through a network communication device to view the detected health indicators. The communication module 7 is a 4G (4^{th} generation mobile communication technology) or 5G (5^{th} generation mobile communication technology) module.

Preferably, the control system 3 further comprises a printer drive module 8, wherein after receiving the detected health indicators, the main control module 5 sends the detected health indicators to a print device 13 for printing through the printer drive module 8.

Preferably, the control system 3 further comprises a forewarning module 9, wherein after receiving the detected health indicators, the main control module 5 compares the detected health indicators with forewarning data, an alarm is sent through the forewarning module 9 when the detected health indicators exceed a range of the forewarning data.

Preferably, the control system 3 further comprises a storage module 10 for storing the detected health indicators received by the main control module 5.

According to the present invention, the detection system capable of detecting the health indicators of the doctors is connected with the medical apparatus. The doctor health monitoring system provided by the present invention is able to monitor, diagnose and treat pulse, blood pressure, body temperature, heart rate, electrocardiogram and other health indicators of the doctors in real time, and is also able to predict the physical health status of the doctors. When a certain health indicator exceeds a forewarning value, a warning device automatically sends an alarm, so as to better protect the physical health of the doctors.

The content not described in detail in the embodiment of the present invention belongs to the prior art known to those skilled in the art.

## Claims

1. A doctor health monitoring system based on a medical apparatus, the doctor health monitoring system comprising the medical apparatus (1), a detection system (2), a control system (3) and an artificial intelligence system (4), wherein:
the detection system (2) is configured to detect health indicators of doctors and send the detected health indicators to the control system (3);
the control system (3) and the artificial intelligence system (14) are integrated within the medical apparatus (1);
the control system (3) is configured to send the detected health indicators from the detection system to a display device (11) for display and transmit the detected health indicators to a cloud server (12) for storage;
the artificial intelligence system (14) is configured to analyze the detected health indicators in the cloud server for obtaining a diagnostic accuracy of the detected health indicators.

2. The doctor health monitoring system according to claim 1, wherein the detection system (2) comprises a pulse sensor module, a blood pressure sensor module, a heart rate sensor module, a body temperature sensor module, and an ECG (electrocardiogram) sensor module, wherein the pulse sensor module, the blood pressure sensor module, the heart rate sensor module, the body temperature sensor module, and the ECG sensor module are configured to detect pulse monitoring data, blood pressure monitoring data, heart rate monitoring data, body temperature monitoring data, and ECG monitoring data of the doctors, respectively.

3. The doctor health monitoring system according to claim 1, wherein the detection system (2) is embedded into the medical apparatus (1) and is configured to collect the health indicators of the doctors through the pulse sensor module, the blood pressure sensor module, the heart rate sensor module, the body temperature sensor module, and the ECG sensor module.

4. The doctor health monitoring system according to claim 1, wherein the control system (3) comprises an MCU (micro controller unit) (4), a main control module (5) and a display module (6), wherein the MCU (4) is configured to read the detected health indicators detected by the detection system in real time and send the detected health indicators to the main control module (5); the main control module (5) is configured to display the detected health indicators on the display device (11) through the display module (6) after receiving the detected health indicators.

5. The doctor health monitoring system according to claim 4, wherein the control system (3) further comprises a communication module (7), wherein after reading the detected health indicators, the MCU (4) sends the detected health indicators to the cloud server through the communication module (7) for storage, and logs in to the cloud server to view the detected health indicators.

6. The doctor health monitoring system according to claim 4, wherein the control system (3) further comprises a printer drive module (8), wherein after receiving the detected health indicators, the main control module (5) sends the detected health indicators to a print device (13) for printing through the printer drive module (8).

7. The doctor health monitoring system according to claim 4, wherein the control system (3) further comprises a forewarning module (9), wherein after receiving the detected health indicators, the main control module (5) compares the detected health indicators with forewarning data, an alarm is sent through the forewarning module (9) when the detected health indicators exceed a range of the forewarning data.

8. The doctor health monitoring system according to claim 4, wherein the control system (3) further comprises a storage module (10) for storing the detected health indicators received by the main control module (5).
